# EUROPEAN PATENT APPLICATION

(11) **EP 2 090 564 A1**
(43) Date of publication of application: **19.08.2009**
(21) Application number: 07830455.7
(22) Date of filing: 24.10.2007
(51) Int. Cl.: C07C 67/08, C07C 69/54, C08F 20/20, G03F 7/027

(54) **ADAMANTANE DERIVATIVE, PROCESS FOR PRODUCTION THEREOF, RESIN COMPOSITION, AND CURED PRODUCT OF THE RESIN COMPOSITION**

(30) Priority: 25.10.2006 JP 2006290242
(71) Applicant: Idemitsu Kosan Co., Ltd., Chiyoda-ku Tokyo 100-8321 (JP)
(72) Inventor: OKADA, Yasunari, Ichihara-shi Chiba 2990193 (JP); YAMANE, Hideki, Ichihara-shi Chiba 2990193 (JP); ITO, Katsuki, Ichihara-shi Chiba 2990193 (JP); MATSUMOTO, Nobuaki, Ichihara-shi Chiba 2990193 (JP)
(74) Representative: Vossius & Partner
(86) International application number: PCT/JP2007/070721
(87) International publication number: WO 2008/050796

(57) **Abstract**

Disclosed herein is an adamantane derivative represented by the following general formula (I): 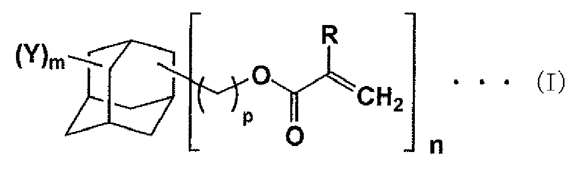 (wherein R represents a hydrogen atom, a fluorine atom, a methyl group or a trifluoromethyl group; Y represents a hydrogen atom, a hydroxyl group, =O formed by two Ys together, a carboxyl group, or a hydrocarbon group having 1 to 20 carbon atoms or a cyclic hydrocarbon group having 3 to 20 carbon atoms; m is 13 and n is 3, or m is 12 and n is 4; and p is an integer of one or more), a production method thereof, a resin composition containing the adamantane derivative, and a cured product thereof. The present invention provides a cured product advantageous as various coating agents, a microlens, a film condenser, a colored composition for a liquid crystal color filter, a pattern organizer formed in nanoimprinting, and the like, having transparency, heat resistance, good mechanical properties, low volume shrinkage, and low coefficient of linear expansion, as well as liquid adamantyl group-containing (meth)acrylates to produce the cured product, the production method thereof, and the resin composition.

## Description

### Technical Field

The present invention relates to a novel adamantane derivative, a production method thereof, a resin composition containing the adamantane derivative, and a cured product thereof, more specifically, to a novel adamantyl group-containing (meth)acrylate which is useful as various coating agents, a microlens, a film condenser, a colored composition for a liquid crystal color filter, a pattern organizer formed in nanoimprinting, and the like in the field of electronic and optical materials, a production method thereof, a resin composition containing the adamantane derivative, and a cured product thereof.

### Background Art

Adamantane is a highly symmetrical and stable compound in which four cyclohexane rings are condensed to form a cage-like structure. The derivative of adamantane is known as a useful raw material for pharmaceuticals, highly functional industrial materials and the like because of its unique functions. For example, attempts to use the adamantane derivative for a substrate of an optical disk, an optical fiber, a lens, and the like are being made because the adamantane derivative has optical properties, heat resistance, and the like (refer to Patent Documents 1 and 2). Further, attempts to use adamantane esters as a raw material for a photoresist resin are also being made by employing its acid sensitivity, resistance to dry etching, transmittance of an ultraviolet light, and the like (refer to Patent Document 3).
In recent years, in the field of electronic and optical materials, investigation is progressing to enhance or improve the performance of optical and electronic parts. Examples of such investigation are improvement of fineness, widening of view angle, and improvement of image quality of a flat panel display using liquid crystal, organic electroluminescence (organic EL) and the like, increasing of brightness, shortening of wavelength, and increasing of whiteness of the light source using an optical semiconductor such as a light-emitting diode (LED) and the like, as well as increasing of frequency of electronic circuit and optical circuit and optical communication.
In addition, semiconductor technology is significantly progressing and electronic devices are rapidly becoming miniaturized, light-weighted, highly performed, and multifunctionalized. In response to this trend, high density and multiple wiring are desired for interconnection substrates.
As the materials for such optical and electronic parts, various thermosetting resins, light curing resins, thermoplastic resins or the like are applied. For example, these resins are used for a microlens, various coating agents, a film condenser, a colored composition for a liquid crystal color filter, and the like.

For example, a microlens array in which many microlenses with convex and concave shapes are disposed on a substrate is used as an optical part for a liquid crystal display, a projector, an image sensor, and the like because it enables the improvement of efficiency of light utilization. Examples of the method for producing a microlens are (1) pattern-exposing and developing a photosensitive resin to form a pattern corresponding to the lens shape followed by melt-flowing the resin, (2) transferring the lens shape to a substrate by dry etching using the melt-flown lens pattern as a mask, (3) coating a glass substrate and the like with a resin composition followed by pressing the resin with a mold having the lens shape and curing the resin as it is. When the photosensitive resin in the above-mentioned method (1) is used, transparency to the exposure wavelength is required. In the above-mentioned etching method (2), resistance to etching is required. In the above-mentioned curing method (3) using a mold, the resin composition is required to be liquid. In addition, as the performance of the microlens itself, transparency, heat resistance, light resistance, and the like are required. For example, as a resin composition for microlens, an irradiation sensitive resin composition containing (meth)acrylate having a tricyclodecane structure is disclosed (refer to Patent Document 4). However, although these (meta)acrylates have a transparency, it is hard to say that the heat resistance of them is sufficient.
A color filter used for a liquid crystal display is formed by successively exposing and developing a colored composition with red, blue and green pigments dispersed in it (a colored resist) and a colored composition with black pigment dispersed in it (a black matrix). In many cases, a polyfunctional acrylate is added in the compositions in order to form each pixel. For example, acrylates such as pentaerythritol and dipentaerythritol are disclosed as a coloring composition for a color filter (refer to Patent Document 5). However, the heat resistance and mechanical strength of these acrylates are not satisfactory.
In addition, various methods are being investigated to form a fine pattern because further improvement in finesse is required in the process of lithography in production of semiconductors. One of these methods is nanoimprint lithography. It is desired that the resin composition for nanoimprint lithography has not only an etching resistance, but also a low volume shrinkage and a low coefficient of linear expansion. The resin composition is also desired to be liquid because it is cured by pressing with a mold.

Patent Document 1: Japanese Patent Laid-Open Publication No. H06-305044
Patent Document 2: Japanese Patent Laid-Open Publication No. H09-302077
Patent Document 3: Japanese Patent Laid-Open Publication No. H04-39665
Patent Document 4: Japanese Patent Laid-Open Publication No. 2005-134440
Patent Document 5: Japanese Patent Laid-Open Publication No. 2006-99033

### Disclosure of the Invention

From the circumstances mentioned above, an object of the present invention is to provide a cured product advantageous as various coating agents, a microlens, a film condenser, a colored composition for a liquid crystal color filter, a pattern organizer formed in nanoimprinting, and the like, excellent in transparency, heat resistance and mechanical properties, and having a low volume shrinkage and a low coefficient of linear expansion, as well as to provide liquid adamantyl group-containing (meth)acrylates to produce the cured product, the production method thereof, and the resin composition containing the same.

As a result of keen examination in order to achieve the above-mentioned objects, the present inventors found that a novel adamantane derivative having a specific structure and a resin composition containing these compounds and its cured product are suitable for the object and that the adamantane derivative can be effectively produced by reacting the corresponding adamantyl group-containing alcohols and (meth)acrylic acids or reactive derivatives thereof and completed the present invention.
Thus, the present invention provides the following (1) to (5).
(1) An adamantane derivative represented by the following general formula (I):

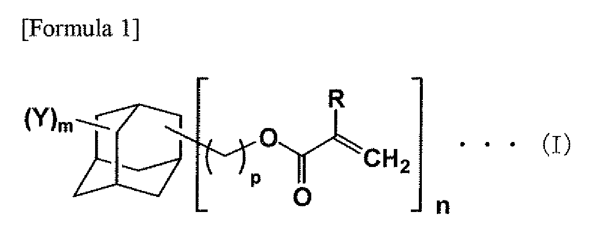

(In the formula, R represents a hydrogen atom, a fluorine atom, a methyl group or a trifluoromethyl group; Y represents a hydrogen atom, a hydroxyl group, =O formed by two Ys together, a carboxyl group, or a hydrocarbon group having 1 to 20 carbon atoms or a cyclic hydrocarbon group having 3 to 20 carbon atoms which may contain a hetero atom; the plural Y may be the same or different from each other; m is 13 and n is 3, or m is 12 and n is 4; and p is an integer of one or more.)
(2) A method for producing an adamantane derivative represented by the following general formula (I): characterized by reacting adamantyl group-containing alcohols represented by the following general formula (II)

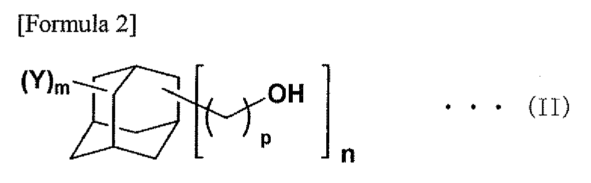

(In the formula, Y is a hydrogen atom, a hydroxyl group, =O formed by two Ys together, a carboxyl group, or a hydrocarbon group having 1 to 20 carbon atoms or a cyclic hydrocarbon group having 3 to 20 carbon atoms which may contain a hetero atom; the plural Ys may be the same or different from each other; m is 13 and n is 3, or m is 12 and n is 4; and p is an integer of 1 or more.)
and (meth)acrylic acids represented by the following general formula (III):

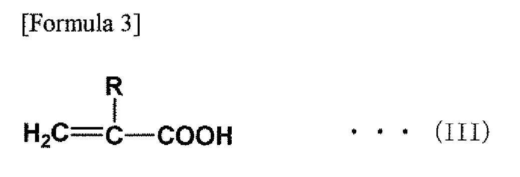

(In the formula, R represents a hydrogen atom, a fluorine atom, a methyl group or a trifluoromethyl group.)
or reactive derivatives thereof.

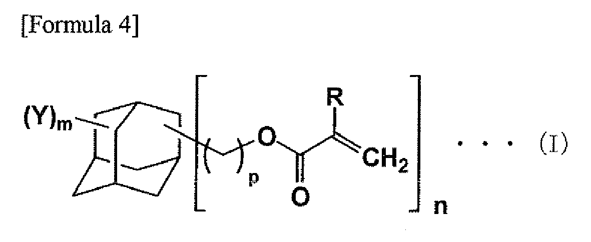

(In the formula, R, Y, m, n, and p are the same as above.)
(3) A resin composition characterized by containing the adamantane derivative described in the above-mentioned (1) and a thermopolymerization initiator or a photopolymerization initiator.
(4) The resin composition described in the above-mentioned (3), characterized by containing 0.01 to 10 mass% of a thermopolymerization initiator or 0.01 to 10 mass% of a photopolymerization initiator relative to the total amount of the resin composition.
(5) A cured product characterized by being obtained by curing the resin composition described in the above-mentioned (3) or (4) by light irradiation or heating.

According to the present invention, a cured product advantageous as various coating agents, a microlens, a film condenser, a colored composition for a liquid crystal color filter, a pattern organizer formed in nanoimprinting, and the like, having excellent in transparency, heat resistance and mechanical properties, and having a low volume shrinkage and a low coefficient of linear expansion, as well as liquid adamantyl group-containing (meth)acrylates to produce the cured product, the production method thereof, and the resin composition containing the same can be provided.

### Best Mode for Carrying Out the Invention

The adamantane derivative of the present invention is a compound represented by the general formula (I). The compounds and their production method will be explained hereinafter.

### Adamantane Derivative

First of all, the adamantane derivative of the present invention is a compound having a structure represented by the following general formula (I):

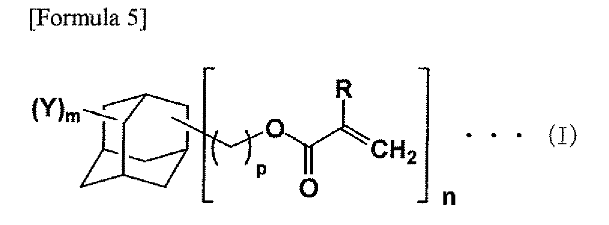

In the general formula (I), R represents a hydrogen atom, a fluorine atom, a methyl group or a trifluoromethyl group; Y represents a hydrogen atom, a hydroxyl group, =O formed by two Ys together, a carboxyl group, or a hydrocarbon group having 1 to 20 carbon atoms or a cyclic hydrocarbon group having 3 to 20 carbon atoms which may contain a hetero atom; the plural Ys may be the same or different from each other; m is 13 and n is 3, or m is 12 and n is 4; and p is an integer of one or more. In the general formula (I), the substituents including (meth)acrylate may be the same or different from each other. Note that "(meth)acrylate" in this specification indicates acrylate or methacrylate.
The hydrocarbon group having 1 to 20 carbon atoms in the above-mentioned Y may be linear or branched and includes, for example, a methyl group, an ethyl group, various propyl groups, various butyl groups, various pentyl groups, various hexyl groups, various heptyl groups, various octyl groups, various nonyl groups, various decyl groups, various dodecyl groups, various tetradodecyl group, various hexadecyl groups, various octadecyl groups, various icosyl groups and the like. The cyclic hydrocarbon group having 3 to 20 carbon atoms includes a cyclopentyl group, a cyclohexyl group, a cyclooctyl group, a cyclododecyl group, an adamantyl group, a group introduced by a lower alkyl group on the ring of the above-mentioned groups, and the like. A hetero atom includes nitrogen, oxygen, and sulfur. The above-mentioned hydrocarbon group and the cyclic hydrocarbon group may be substituted by a hydroxyl group or the like.
The above-mentioned p is preferably an integer of 1 to 10, more preferably 1 to 5, and especially preferably 1 to 3, from a viewpoint of maintenance of heat resistance, strength, and low coefficient of linear expansion.

Examples of the preferred compound represented by the above-mentioned general formula (I) include adamantane-1,3,5-trimethanol triacrylate, adamantane-1,3,5,-triethanol triacrylate, adamantane-1,3,5-tripropanol triacrylate, adamantane-1,3,5,7-tetramethanol tetraacrylate, adamantan-1,3,5,7-tetraethanol tetraacrylate, adamantane-1,3,5,7-tetrapropanol tetraacrylate, and a compound in which the acrylate moiety of these compounds is replaced by α-fluoroacrylate, methacrylate, or α-trifluoromethylacrylate, and the like.

### Production Method of Adamantane Derivative

Next, the production method of the adamantane derivative of the present invention will be explained.
The adamantane derivative represented by the following general formula (I):

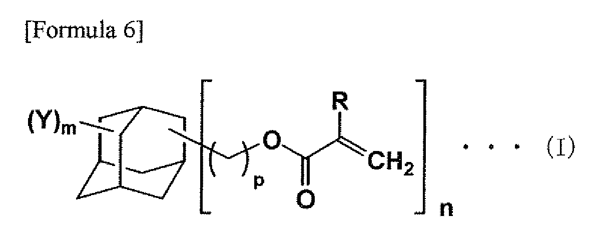

(wherein R, Y, m, n, and p are the same as above.)
can be synthesized by reacting the adamantyl group-containing alcohols represented by the following general formula (II):

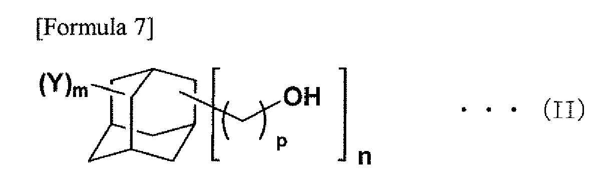

(wherein Y, m, n, and p are the same as above.)
and (meth)acrylic acids represented by the following general formula (III):

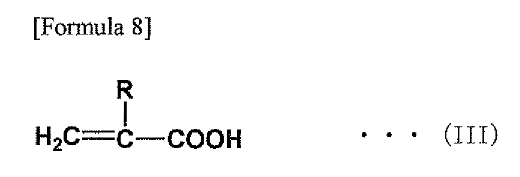

(wherein R is the same as above.)
or reactive derivatives thereof Specifically, the adamantane derivative can be synthesized by esterification reacting the above-mentioned adamantyl group-containing alcohols and (meth)acrylic acids or reactive derivatives thereof using a usually known azeotropic dehydration method, acid halide method, or ester exchange method.

Examples of the adamantyl group-containing alcohols represented by the general formula (II) include adamantane-1,3,5-trimethanol, adamantane-1,3,5-triethanol, adamantane-1,3,5-tripropanol, adamantane-1,3,5,7-tetramethanol, adamantane- 1,3,5,7-tetraethanol, adamantane-1,3,5,7-tetrapropanol, and the like.

In the case of the above-mentioned azeotropic dehydration method, examples of (meth)acrylic acids represented by the general formula (III) or reactive derivatives thereof include acrylic acid, methacrylic acid, α-trifluoromethylacrylic acid, α-fluoroacrylic acid, and acid anhydrides such as acrylic anhydride, methacrylic anhydride, α-trifluoromethylacrylic anhydride,α-fluoroacrylic anhydride and the like, and others. In the case of the above-mentioned acid halide method, examples include acid halides such as acrylic chloride, methacrylic chloride, α-trifluoromethylacrylic chloride, α-fluoroacrylic chloride and the like, and others. In the case of the above-mentioned ester exchange method, examples include lower alkyl esters such as methyl acrylate, ethyl acrylate, propyl acrylate, and compounds in which the acrylic acid moiety of these compounds is replaced by methacrylic acid, α-trifluoromethylacrylic acid, α-fluoroacrylic acid and the like, and others.
The amount of the (meth)acrylic acids represented by the general formula (III) or the reactive derivatives thereof to be used is preferably about 1 to 3 times the stoichiometric quantity relative to the above-mentioned adamantyl group-containing alcohols.

Next, the above-mentioned azeotropic dehydration method will be explained.
In this reaction, a catalyst may be used as needed. Examples of the catalyst include sulfuric acid, p-toluenesulfonic acid, and the like. These catalysts may be used solely or in combination of two or more kinds. The amount of the catalyst to be used is usually 0.01 to 20 mol%, preferably 0.05 to 10 mol%, relative to the above-mentioned adamantyl group-containing alcohols as a raw material.
In this reaction, a solvent may be used as needed. The solvent is selected so that the solubility of the above-mentioned adamantyl group-containing alcohols to the solvent is usually 0.5 mass% or more, preferably 5 mass% or more, at the reaction temperature. The amount of the solvent is selected so that the concentration of the above-mentioned adamantyl group-containing alcohols is usually 0.5 mass% or more, preferably 5 mass% or more. In this reaction, although the above-mentioned adamantyl group-containing alcohols may be suspended in the solvent, it is preferable that the alcohols are dissolved in the solvent. Specific examples of the solvent used include nonane, decane, undecane, cyclohexane, methylcyclohexane, ethylcyclohexane, toluene, xylene, N,N-dimethyl formamide (DMF), N-methyl-2-pyrrolidone (NMP), N,N-dimethylacetamide (DMAc), dimethylsulfoxide (DMSO), and mixed solvents thereof. These solvents may be used solely or in a mixture of two or more kinds.
In this reaction, a polymerization inhibitor may be added as needed. Examples of the polymerization inhibitor include hydroquinone, methoquinone, phenothiazine, methoxyphenothiazine and the like. These polymerization inhibitors may be used solely or in a mixture of two or more kinds. The amount of the polymerization inhibitor to be used is usually 10 to 10,000 mass ppm, preferably 50 to 5,000 mass ppm, relative to the above-mentioned (meth)acrylic acids and reactive derivatives thereof

Reaction temperature is usually 50 to 200°C. If the temperature is 50°C or higher, the reaction rate is adequate and reaction time is not excessively long. If the temperature is 200°C or lower, side reaction can be suppressed and discoloration is not severe. Preferably the reaction temperature is 100 to 180°C.
Reaction pressure applied is usually in a range of 0.01 to 10 MPa in absolute pressure. In this range, there is no need of special equipment for a safety problem. Therefore, this method is industrially useful. The reaction pressure is preferably normal pressure to 1 MPa.
Although reaction time cannot be determined uniformly because it depends on a type and amount of the catalyst, reaction temperature, and the like, it is usually in a range of 1 to 48 hours, preferably 1 to 24 hours.
In this reaction, it is desirable to make favor for the product formation system in the equilibrium reaction by removing the water generated by the reaction from the reaction mixture.

Next, the above-mentioned acid halide method will be explained.
In this reaction, a base may be used as an acid capture agent to capture acid generated by the reaction. Examples of the base include an organic amine such as triethylamine, tributylamine, pyridine, dimethylaminopyridine and the like, and an inorganic base such as sodium hydroxide, potassium hydroxide, sodium carbonate, potassium carbonate, sodium phosphate, potassium phosphate and the like. These bases may be used solely or in a mixture of two or more kinds. The ratio of the base to be used is usually 0.5 to 5 times, preferably 1 to 3 times the stoichiometric quantity relative to the adamantyl group-containing alcohols represented by the general formula (II).
In this reaction, a solvent may be used as needed. The solvent is selected so that the solubility of the above-mentioned adamantyl group-containing alcohols to the solvent is usually 0.5 mass% or more, preferably 5 mass% or more, at the reaction temperature. The amount of the solvent is selected so that the concentration of the above-mentioned adamantyl group-containing alcohols is usually 0.5 mass% or more, preferably 5 mass% or more. In this reaction, although the above-mentioned adamantyl group-containing alchohols may be suspended in the solvent, it is preferable that the alcohols are dissolved in the solvent. Specific examples of the solvent used include hexane, heptane, cyclohexane, toluene, DMF, NMP, DMAc, DMSO, diethylether, tetrahydrofuran (THF), ethyl acetate, dichloromethane, chloroform and the like. These solvents may be used solely or in a mixture of two or more kinds.
In this reaction, a polymerization inhibitor may be added as needed. Examples of the polymerization inhibitor include hydroquinone, methoquinone, phenothiazine, methoxyphenothiazine and the like. These polymerization inhibitors may be used solely or in a mixture of two or more kinds. The amount of the polymerization inhibitor to be used is usually 10 to 10,000 mass ppm, preferably 50 to 5,000 mass ppm, relative to the above-mentioned (meth)acrylic acids and reactive derivatives thereof.

Reaction temperature is usually -50 to 100°C. If the temperature is -50°C or higher, there is no need of special equipment and therefore, this method is industrially useful. If the temperature is 100°C or lower, side reaction can be suppressed and discoloration is not severe. Preferably the reaction temperature is 0 to 50°C.
Reaction pressure applied is usually in a range of 0.01 to 10 MPa in absolute pressure. In this range, there is no need of special equipment for a safety problem. Therefore, this method is industrially useful. The reaction pressure is preferably normal pressure to 1 MPa.
Although reaction time cannot be determined uniformly because it depends on a type and amount of the catalyst, reaction temperature, and the like, it is usually in a range of 5 minutes to 24 hours, preferably 5 minutes to 10 hours.

Next, the above-mentioned ester exchange method will be explained.
In this reaction, a catalyst may be used as needed. Examples of the catalyst include sulfuric acid, p-toluenesulfonic acid, sodium methoxide, sodium t-butoxide, potassium methoxide, potassium t-butoxide and the like. These catalysts may be used solely or in combination of two or more kinds. The amount of the catalyst to be used is usually 0.01 to 50 mol%, preferably 0.5 to 20 mol%, relative to the above-mentioned adamantyl group-containing alcohols as a raw material.
In this reaction, a solvent may be used as needed. The solvent is selected so that the solubility of the above-mentioned adamantyl group-containing alcohols to the solvent is usually 0.5 mass% or more, preferably 5 mass% or more at the reaction temperature. The amount of the solvent is selected so that the concentration of the above-mentioned adamantyl group-containing alcohols is usually 0.5 mass% or more, preferably 5 mass% or more. In this reaction, although the above-mentioned adamantyl group-containing alchohols may be suspended in the solvent, it is preferable that the alcohols are dissolved in the solvent. Specific examples of the solvent used include nonane, decane, undecane, cyclohexane, methylcyclohexane, ethylcyclohexane, toluene, xylene, DMF, NMP, DMAc, DMSO and the like. These solvents may be used solely or in a mixture of two or more kinds.
In this reaction, a polymerization inhibitor may be added as needed. Examples of the polymerization inhibitor include hydroquinone, methoquinone, phenothiazine, methoxyphenothiazine and the like. These polymerization inhibitors may be used solely or in a mixture of two or more kinds. The amount of the polymerization inhibitor to be used is usually 10 to 10,000 mass ppm, preferably 50 to 5,000 mass ppm, relative to the above-mentioned (meth)acrylic acids and reactive derivatives thereof

Reaction temperature is usually 0 to 200°C. If the temperature is 0°C or higher, the reaction rate is adequate and reaction time is not excessively long. If the temperature is 200°C or lower, side reaction can be suppressed and discoloration is not severe. Preferably the reaction temperature is 25 to 180°C.
Reaction pressure applied is usually in a range of 0.01 to 10 MPa in absolute pressure. In this range, there is no need of special equipment for a safety problem. Therefore, this method is industrially useful. The reaction pressure is preferably normal pressure to 1 MPa.
Although reaction time cannot be determined uniformly because it depends on a type and amount of the catalyst, reaction temperature, and the like, it is usually in a range of 1 to 48 hours, preferably 1 to 24 hours.
In this reaction, it is desirable to make favor for the product formation system in the equilibrium reaction by removing the lower alkyl alcohols generated by the ester exchange from the reaction system.

As a purification method of the objective reaction product, distillation, crystallization, column separation, and the like are applicable and may be selected according to the property of the product and the kind of the impurities.

### Resin Composition and Cured Product Thereof

The resin composition of the present invention can be obtained by mixing the above-mentioned adamantyl group-containing (meth)acrylate with a thermopolymerization initiator or a photopolymerization initiator. The resin composition can be cured by thermal curing or photocuring by ultraviolet (UV) irradiation and the like, after forming into desired shape by injection into a molding die or coating.
The amount of the above-mentioned adamantyl group-containing (meth)acrylate to be used is usually 5 to 99.9 mass%, preferably 10 to 99.9 mass%, relative to the total amount of the composition. These acrylates may be used solely or in combination of two or more kinds.
In this case, the above-mentioned adamantyl group-containing (meth)acrylate may be used solely or the composition may contain a compound having a polymerizable unsaturated group as another polymerizable monomer, as long as it does not adversely affect the heat resistance or mechanical properties, and the like. Examples of such another polymerizable monomer include methyl (meth)acrylate, ethyl (meth)acrylate, propyl (meth)acrylate, butyl (meth)acrylate, adamantyl (meth)acrylate, benzyl (meth)acrylate, ethyleneglycol di(meth)acrylate, 1,3-propanediol di(meth)acrylate, 1,4-butanediol di(meth)acrylate, tricyclodecanedimethanol di(meth)acrylate, adamantane-1,3-diol di(meth)acrylate, adamantane-1,3-dimethanol di(meth)acrylate, adamantane-1,3-diethanol di(meth)acrylate, 5,7-dimethyladamantane-1,3-diol di(meth)acrylate, 5,7-dimethyl adamantane-1,3-dimethanol di(meth)acrylate, 5,7-dimethyladamantane-1,3-diethanol di(meth)acrylate, pentaerythritol tetraacrylate, dipentaerythritol hexaacrylate and the like.
The amount of these another polymerizable monomers to be used is preferably 0 to 95 mass%, more preferably 0 to 90 mass%, relative to the total amount of the composition. These monomers may be used solely or in combination of two or more kinds.

A polymerization initiator necessary for curing is contained in the above-mentioned resin composition. In the case of heat curing, a thermopolymerization initiator is contained. In the case of curing by light irradiation, a photopolymerization initiator is contained.
Examples of the thermopolymerization initiator include organic peroxide such as benzoyl peroxide, methyl ethyl ketone peroxide, methyl isobutyl ketone peroxide, cumene hydroperoxide, t-butyl hydroperoxide and the like, azo-type initiator such as azobisisobutylonitrile and the like, and others.
Examples of the photopolymerization initiator include acetophenones, benzophenones, benzils, benzoin ethers, benzil diketals, thioxanthones, acylphosphine oxides, acylphosphinic acid esters, aromatic diazonium salts, aromatic sulfonium salts, aromatic iodonium salts, aromatic iodosyl salts, aromatic sulfoxionium salts, metallocene compounds and the like.
The amount of these polymerization initiators to be used is usually 0.01 to 10 mass%, preferably 0.05 to 5 mass%, relative to the total amount of the composition. These polymerization initiators may be used solely or in combination of two or more kinds.

The cured product of the present invention can be obtained by heating or photocuring the above-mentioned resin composition.
The thermocuring temperature is usually 30 to 200°C, preferably 50 to 150°C. In the case of photocuring, the cured product can be obtained by irradiation with an active light such as an ultraviolet ray and the like. Although the irradiation intensity is optional because it is determined by the kind of the monomer and polymerization initiator, film thickness of the cured product, and the like, it is usually 100 to 5,000 mJ/cm², more preferably 500 to 4,000 mJ/cm².
The cured product of the resin composition obtained by the present invention is excellent in transparency, optical property such as (long-term) light resistance and the like, and heat resistance, and has good mechanical properties, a low coefficient of linear expansion and a low volume shrinkage. Therefore, the cured product can be advantageously used as various coating agents, a lens, a microlens, a film condenser, a colored composition for a liquid crystal color filter, a pattern organizer formed in nanoimprinting, and the like.

### Examples

Hereinafter, the examples and comparative examples of the present invention will be explained more specifically. The present invention is not at all limited by these examples.

### Evaluation of the Physical Properties

The cured product of the resin composition obtained in the following examples and comparative examples was evaluated as follows.

### (1) Decomposition Temperature

Decomposition temperature was measured by raising the temperature of 10 mg of a sample at a rate of 10 °C/minute under nitrogen atmosphere using a differential scanning calorimeter (DSC-7 manufactured by PerkinElmer Inc.). The decomposition temperature was defined as the temperature at the time when 5 mass% was decreased relative to the whole mass.

### (2) Total LightTransmittance

Total light transmittance was measured according to JIS K7105. SM Color Computer manufactured by Suga Test Instruments Co., Ltd. was used as the measuring apparatus.

### (3) Bending Test

Bending strength was measured according to JIS K7171. Universal Materials Testing Machine 5582 manufactured by Instron Corporation was used as the measuring apparatus. The sample size was 40 × 25 × 1 mm thick. Distance between the supporting points was 16 mm. Bending speed was 1 mm/minute.

### (4) Volume shrinkage

A specific gravity of the resin composition before curing and a specific gravity of the cured product of the resin composition were measured at 25°C. Volume shrinkage was calculated from a difference between the specific gravities of the resin compositions before and after curing.

### (5) Coefficient of linear expansion

Coefficient of linear expansion was measured using TMA 8310 apparatus manufactured by Rigaku Corporation. Sample size was 10 × 10 × 1 mm thick. Measurement was done under nitrogen stream loading 20 mN, at a temperature raising rate of 5 °C/minute from 25 to 180°C.

### Example 1

### Synthesis of Adamantane-1,3,5-trimethanol Triacrylte

A 1,000 mL four-necked flask equipped with a reflux condenser with a Dean-Stark apparatus, a stirrer, a thermometer, and an air inlet tube was charged with 50 g (0.22 mol) of adamantane-1,3,5-trimethanol, 55.7 g (0.77 mol) of acrylic acid, 500 mL of toluene, 1.11 g of 98 mass% sulfuric acid, and 0.06 g of methoquinone and heated under reflux in an oil bath at 130°C for 3 hours. The reaction was carried out removing the water generated by the progress of the reaction out of the reaction system. After that, the reaction mixture was allowed to cool to the room temperature and then transferred to a separatory funnel. Then, the reaction mixture was washed with 250 mL of aqueous 5 mass% sodium chloride solution. The organic layer was washed with 250 mL of aqueous 3 mass% sodium phosphate solution and then with 250 mL of 5 mass% aqueous sodium chloride solution. The washed organic layer was then dehydrated by anhydrous magnesium sulfate followed by removing magnesium sulfate by filtration. The solvent was evaporated from the filtrate to obtain a liquid crude product, which was then dissolved into 600 mL of n-hexane. To this solution 7 g of silica gel was added. After stirring the mixture for 30 minutes, silica gel was removed by filtration. From the filtrate n-hexane was evaporated to obtain 62.8 g of the objective adamantane-1,3,5-trimethanol triacrylate as a colorless, transparent liquid (yield: 81 %). ¹H-NNM spectrum and ¹³C-NMR spectrum were measured for the obtained compound using JNM-ECA500 manufactured by JEOL Ltd. as a measurement apparatus and chloroform as a solvent at a room temperature condition.

Nuclear Magnetic Resonance Spectroscopy (NMR) (solvent: chloroform-d)
¹H-NMR (500MHz): 1.31-1.58 (m, 13H), 3.74 (s, 6H), 5.77 (d, 3H), 6.06 (dd, 3H), 6.33 (d, 3H)
¹³C-NMR (125MHz): 27.8, 33.6, 36.1, 38.6, 73.4, 128.5, 130.4, 166.2

### Example 2

To 10 g of adamantane-1,3,5-trimethanol triacrylate obtained in Example 1, 0.1 g of benzoin isobutyl ether was added as a photopolymerization initiator. After mixing well, the mixture was degassed under vacuum to obtain a resin composition. The obtained resin composition was cast into a glass cell and irradiated with a mercury lamp at 3,000 mJ/cm² to obtain a cured product of 1 mm thickness. Physical properties of the resultant cured product are shown in Table 1.

### Example 3

To a mixture of 7 g of adamantane-1,3,5-trimethanol triacrylate obtained in Example 1 and 3 g of adamantane-1,3-dimethanol diacrylate, 0.1 g of benzoin isobutyl ether was added as a photopolymerization initiator. After mixing well, the mixture was degassed under vacuum to obtain a resin composition. The obtained resin composition was cast into a glass cell and irradiated with a mercury lamp at 3,000 mJ/cm² to obtain a cured product of 1 mm thickness. Physical properties of the resultant cured product are shown in Table 1.

### Comparative Example 1

Except that adamantane-1,3,5-trimethanol triacrylate was replaced by tricyclodecanedimethanol diacrylate, a cured product of 1 mm thickness was obtained by the similar method to that in Example 2. Physical properties of the resultant cured product are shown in Table 1. It is shown that this cured product has lower decomposition temperature and larger coefficient of linear expansion compared with Examples 2 and 3.

### Comparative Example 2

Except that adamantane-1,3,5-trimethanol triacrylate was replaced by pentaerythritol tetraacrylate, a cure product of 1 mm thickness was obtained by the similar method to that in Example 2. Physical properties of the cure product are shown in Table 1. It is shown that this cured product has a smaller bending strength and larger volume shrinkage compared with Examples 2 and 3. The coefficient of linear expansion could not be measured up to 180°C because of insufficient heat resistance.

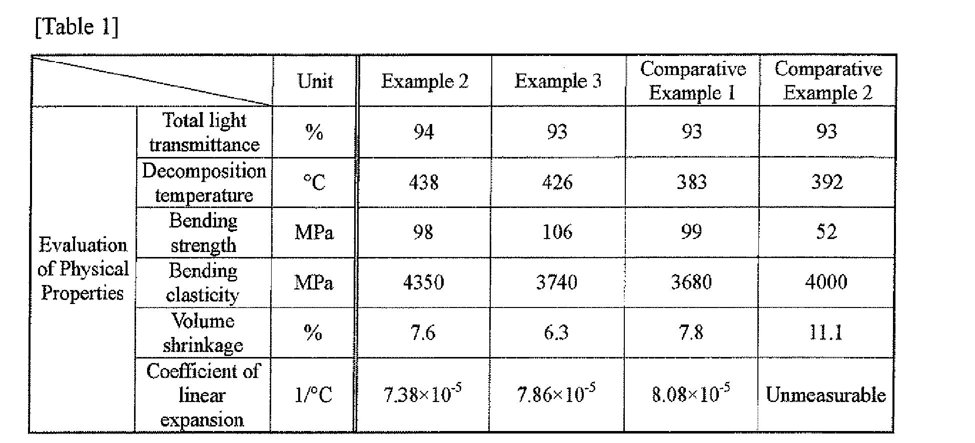

### Industrial Applicability

The adamantane derivative of the present invention and the resin composition containing the same provide a cured product having transparency, heat resistance, good mechanical properties, low coefficient of linear expansion and low volume shrinkage and can be used advantageously as various coating agents, a microlens, a film condenser, a colored composition for a liquid crystal color filter, a pattern organizer formed in nanoimprinting, and the like in the field of electronic and optical materials. In addition, the adamantane derivative of the present invention can be produced efficiently.

## Claims

1. An adamantane derivative represented by the following general formula (I): 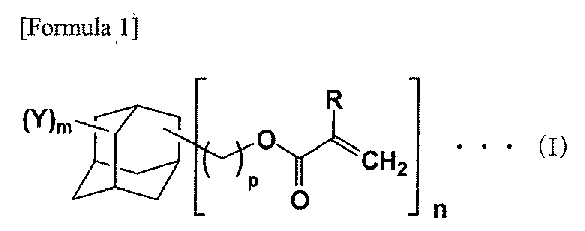 wherein R represents a hydrogen atom, a fluorine atom, a methyl group or a trifluoromethyl group; Y represents a hydrogen atom, a hydroxyl group, =O formed by two Ys together, a carboxyl group, or a hydrocarbon group having 1 to 20 carbon atoms or a cyclic hydrocarbon group having 3 to 20 carbon atoms which may contain a hetero atom; a plurality of Ys may be the same or different from each other; m is 13 and n is 3, or m is 12 and n is 4; and p is an integer of one or more.

2. A method for producing an adamantane derivative represented by the following general formula (I): 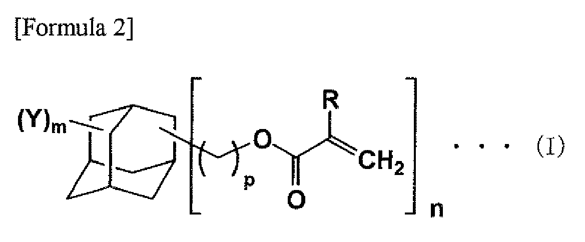 wherein R represents a hydrogen atom, a fluorine atom, a methyl group or a trifluoromethyl group; Y represents a hydrogen atom, a hydroxyl group, =O formed by two Ys together, a carboxyl group, or a hydrocarbon group having 1 to 20 carbon atoms or a cyclic hydrocarbon group having 3 to 20 carbon atoms which may contain a hetero atom; a plurality of Ys may be the same or different from each other; m is 13 and n is 3, or m is 12 and n is 4; and p is an integer of one or more,
comprising reacting adamantyl group-containing alcohols represented by the following general formula (II): 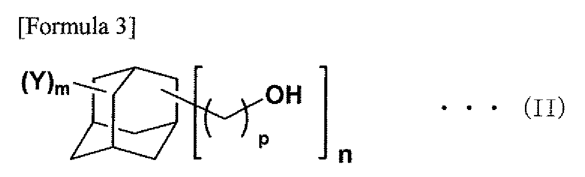 wherein Y, m, n, and p are the same as above;
and (meth)acrylic acids represented by the following general formula (III): 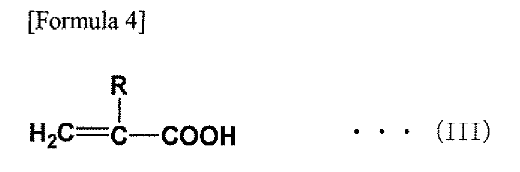 wherein R is the same as above, or reactive derivatives thereof

3. A resin composition containing the adamantane derivative according to claim 1 and a thermopolymerization initiator or a photopolymerization initiator.

4. The resin composition according to claim 3, wherein the resin composition contains 0.01 to 10 mass% of a thermopolymerization initiator or 0.01 to 10 mass% of a photopolymerization initiator relative to the total amount of the resin composition.

5. A cured product obtained by curing the resin composition according to claim 3 or 4 by light irradiation or heating.
